(19) Europäisches Patentamt
European Patent Office
Office européen des brevets

(11) Publication number: **0 109 074**
**A1**

# (12) EUROPEAN PATENT APPLICATION

(21) Application number: **83111309.7**

(22) Date of filing: **12.11.83**

(51) Int. Cl.³: **C 07 C 103/52**, C 07 C 103/54, C 07 C 101/24, C 07 G 7/00 // A61K37/02, A61K7/06

(30) Priority: **16.11.82 US 442236**

(43) Date of publication of application: **23.05.84 Bulletin 84/21**

(84) Designated Contracting States: **DE GB**

(71) Applicant: **INOLEX CHEMICAL COMPANY, Jackson and Swan Streets, Philadelphia Pennsylvania 19148-3497 (US)**

(72) Inventor: **Massuda, David, 1513 Willow Lane, Mt. Prospect, Ill. (US)**

(74) Representative: **Reitzner, Bruno, Dr., Patentanwälte Dipl.-Ing. R. Splanemann Dr. B. Reitzner Tal 13, D-8000 München 2 (DE)**

## (54) Quarternary ammonium derivatives of amino acid units.

(57) A water-soluble, cationic, quaternary ammonium compound is prepared comprising a lipophilic end group, a linking structure derived from a epihalohydrin, and a hydrophilic end segment containing from 1 to 500 amino acid units. Preferably, the hydrophilic end segment is a polypeptide end segment having 3-30 amino acid units and is derived from a protein hydrolysate.

EP 0 109 074 A1

INOLEX CHEMICAL COMPANY
Jackson and Swan Streets
Philadelphia, Pennsylvania 19148-3497
U S A

0109074



# QUATERNARY AMMONIUM DERIVATIVES OF AMINO ACID UNITS

## Technical Field

This invention relates to a water-soluble cationic quaternary ammonium derivative of one or more amino acid units, a method for preparing same, and its use as a surface-active agent. More particularly, the compound comprises a cationic long chain end segment, a linking structure derived from epihalohydrin, and a hydrophilic end segment containing from 1 to 500 amino acid units. Preferably, the hydrophilic end segment is polypeptide derivative obtained from protein hydrolysis; and the description herein is primarily of this preferred embodiment.

## Background Art

Cationic quaternary ammonium derivatives of amino acid units, and particularly of polypeptides, are desirable for use as conditioners, as surfactants in cosmetics and pharmaceuticals applied topically to skin and hair, and in household products that come in contact with the user's skin. Preferably, they should be water-soluble, have a bland odor and be non-irritating to the skin.

Polypeptide compounds obtained from hydrolyzed protein are usually converted into anionic surface-active agents prepared by treating a protein hydrolysate with an acyl chloride in the presence of alkali according to the well-known Schotten-Baumann reaction. These materials are well known in the art and are commercially available. However, little is known about cationic polypeptide compounds, more particularly, water-soluble quaternary ammonium salts of polypeptides having surface-active properties.

The Schotten-Baumann reaction for preparing polypeptide derivatives from protein hydrolysates has some inherent drawbacks. These drawbacks include the high cost of the fatty acid chloride starting material and an incomplete reaction due to partial hydrolysis of the acid chloride resulting in a low yield value of between about 25 to about 50 weight percent. Hydrolysis of fatty acid chlorides also results in the formation of salts of fatty acids that are difficult and costly to remove. The product fatty acid hydrolyzed protein condensate is also sensitive to calcium and magnesium salts resulting in flocculant precipitates that cause turbidity. Consequently, only protein hydrolysates having a short range of about 3 to about 6 amino acid units in a polypeptide chain may be used.

French patent No. 1,149,161 to Bolle et al. teaches that quaternary ammonium derivatives of polypeptides may be prepared from protein hydrolysates in a two-step reaction using chloracetyl chloride to form an acetyl linking agent on the polypeptide in the first step. In the second step, the polypeptide derivative is reacted with a fatty tertiary amine to form a quaternary ammonium salt of polypeptide. However chloracetyl chloride is corrosive, toxic and a highly irritating lachrymator thereby making it difficult to handle. The material is also expensive, thereby making the process costly.

Japanese patents Nos. 79 08 688 and 79 08 728, both to Yanagawa et al., teach the preparation of cationic quaternary ammonium derivatives of polypeptides from collagen protein hydrolysates using a 2-hydroxy-1,3-propylene linking group between the protein hydrolysate radical and the quaternerized amino group. The compounds are non-foaming; and in

order to use the cationic compounds in cosmetic shampoo and dishwashing detergents, the compounds must be combined with conventional surface-active agents.

For use in cosmetics, household and pharmaceutical compositions, a quaternary ammonium derivative of polypeptide prepared from protein hydrolysates should preferably be readily soluble in water and in water-alcohol solutions for easy incorporation into typical emulsion systems known in the art. Ideally, a quaternary ammonium derivative of polypeptide should possess surface-active properties particularly suitable for foam-generating cleansing products and conditioners, thereby avoiding the need for auxiliary foaming and foam-stabilizing agents. Thus, a cationic quaternary ammonium derivative of polypeptide possessing the desirable substantivity characteristic of proteinaceous compounds to hair and skin and the conditioning properties associated with cosmetically useful lipophilic compounds that is also capable of functioning as a surface-active agent would be both novel and useful in the art.

A cationic derivative of proteins should also be produceable in a high yield with little or no loss of protein hydrolysate to unuseable by-products.

## Brief Summary of the Invention

The foregoing desired results are achieved in a water-soluble, cationic, quaternary ammonium derivative of amino acid units which comprises a lipophilic end group, a linking structure derived from an epihalohydrin, and a hydrophilic end segment containing from 1 to 500 amino acid units. Preferably, the hydrophilic end segment is a polypeptide end segment having 3-30 amino acid units and is derived from a protein hydrolysate.

Specifically, a water-soluble cationic quaternary ammonium derivative of polypeptide is one in which all the chain terminal amine groups of the amino acid units in the polypeptide chain are completely reacted. Briefly described, the quaternary ammonium derivative of polypeptide of this invention comprises a cationic lipophilic end group, a linking structure derived from an epihalohydrin, and a hydrophilic polypeptide derivative end group comprising a protein hydrolysate.

The compounds of this invention are represented by the following formula:

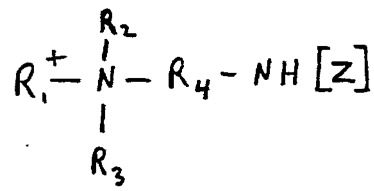

wherein $R_1$ is selected from at least one $C_{12}$ to $C_{22}$ alkyl radical and carboxamide radicals of the formula $R_5-\overset{O}{\overset{\|}{C}}-NH-R_6$ - wherein $R_5$ is selected from at least one $C_{12}$-$C_{22}$ alkyl radical and $R_6$ is selected from at least one $C_2$-$C_3$ alkylene radical, where $R_2$ is at least one $C_1$ to $C_4$ alkyl radical and $R_3$ is selected from the group consisting of at least one $C_1$ to $C_4$ radical and at least one $C_{12}$ to $C_{22}$ alkyl radical; $-R_4-$ is a linking structure derived from an epihalohydrin where the halogen atom comprises chlorine or bromine; and -NH[Z] is an amino acid segment containing from 1 to 500 amino acid units. Preferably -NH[Z] is a polypeptide segment having from 3 to 30 amino acid units.

Briefly described, the method of preparing the preferred cationic quaternary ammonium derivative

of polypeptide comprises the steps of (a) reacting a lipophilic compound of the class consisting of tertiary amines and salts thereof, with an epihalohydrin in the presence of water; and (b) reacting the aqueous reaction mixture of step (a) with an aqueous hydrolyzed protein.

In a broader aspect of the method of this invention, a single amino acid or a synthetic chain of amino acids may be used in place of the hydrolyzed protein. The amino acids may be biological or non-biological amino acids.

The compounds of this invention are easily prepared in high yield and may be used without further purification. They are soluble in water in all proportions, give clear solutions, and do not precipitate on standing thereby showing a high tolerance for electrolytic salts.

The compounds also possess desirable surface-active foaming properties, are substantive to skin and hair, have some antimicrobial activity, and are substantially non-irritating to the skin. Further, the compounds may be prepared from inexpensive protein sources, having a low sulfur content, to achieve products having a desirable light color and bland odor.

A particular advantage of this invention is that quaternary ammonium derivatives of polypeptide attached to a long chain carboxamide end group can be prepared in an aqueous medium.

Thus, the compounds of this invention are useful as fatty conditioners, surface-active foaming agents, proteinaceous skin-protective agents and as cationic anti-microbial agents for cosmetic, pharmaceutical and household products. Further, beneficial advantages of the quaternary ammonium

derivatives of the polypeptides of this invention will become apparent to those skilled in the art from the detailed description of the invention, and the examples which follow.

<u>Detailed Description of the Invention</u>

In the preparation of the compounds of this invention, the long chain compounds to be reacted with the linking structure are preferably selected from the class of tertiary amines and their salts, containing at least one $C_{12}$ to $C_{22}$ alkyl or carboxamide radical, more preferably a $C_{12}$ to $C_{14}$ carboxamide radical in the fatty chain. Exemplary long chain compounds useful in this invention are illustrated, without limitation, by N,N-dimethyldodecylamine, N,N-dimethyltetradecylamine, N,N-dimethyldodecamidopropylamine, N,N-dimethylcocamidopropylamine, and N,N-dimethyltetradecamidopropyl amine.

The linking structure is preferably derived from an epihalohydrin such as epichlorohydrin (1-chloro-2, 3-epoxypropane) or epibromohydrin (1-bromo-2,3- epoxypropane). The preferred linking structure of this invention is derived from epichlorohydrin and is represented by

$-CH_2-\underset{OH}{\underset{|}{CH}}-CH_2-$, or the 2-hydroxy-1,3-propylene group.

The hydrophilic polypeptide material useful as a reactant in the preferred aspect this invention may be derived from hydrolyzed animal and vegetable protein. Preferably, the polypeptide materials are derived from the hydrolysis of collagen-containing animal protein having a low sulfur content using well-known enzymic processes and chemical processes involving either basic or acidic hydrolysis agents. Suitable collagen-containing proteins include scrap

leather, hides, skins, fatty tissues, and pigs' feet. Preferably, water-soluble polypeptides are obtained from hide-derived collagen.

For purposes of illustrating this invention, polypeptide material derived from collagen protein hydrolysate will be used. This material has from about 3 to about 30 amino acid units in a polypeptide chain and a Formol Nitrogen Value in the range of about 8 to about 17, as determined by the conventional Formol Nitrogen Test. The preparation of a particularly suitable polypeptide material by the enzymic hydrolysis of animal protein is described in detail in U.S. Patent No. 3,738,913 to Johnsen, et al., the disclosure of which is incorporated herein by reference. Polypeptide material prepared from leather scraps hydrolyzed with calcium hydroxide is also particularly suitable.

It is to be understood that although the method of preparing a quaternary ammonium derivative of polypeptide is described herein with relation to collagen-derived polypeptides, it is not so limited and may be applied to different sources of protein hydrolysate. Alternate protein hydrolysate sources include, without limitation, dairy proteins, such as casein, vegetable proteins, such as soybean flakes, albuminous proteins, such as gelatin and blood, and hard keratins, such as horn, hair, and hooves. In addition, single amino acids may be used as reactants.

In accordance with one aspect of this invention, an illustrative cationic quaternary derivative of polypeptide is prepared by first reacting a water-soluble salt of the tertiary amine compound (I) with an equimolar amount of an epihalohydrin (II) in water as shown in equation (1) to produce mainly a quaternary nitrogen compound

(III). In a second step, shown in equation (2), the epi-form (IV) of compound (III) is made by adding alkali and is then reacted with protein hydrolysate (V) to form a cationic quaternary derivative of polypeptide (VI).

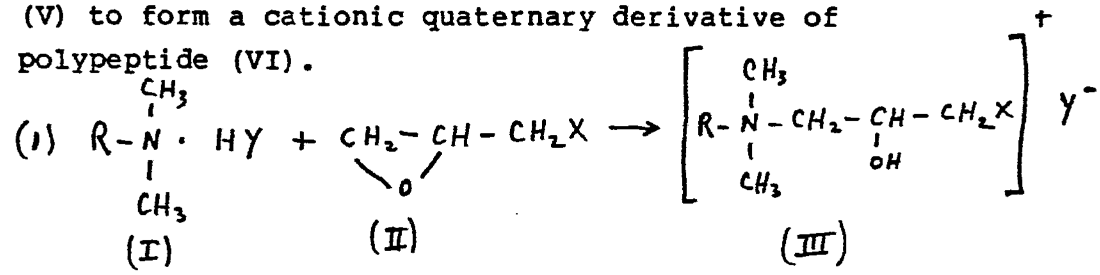

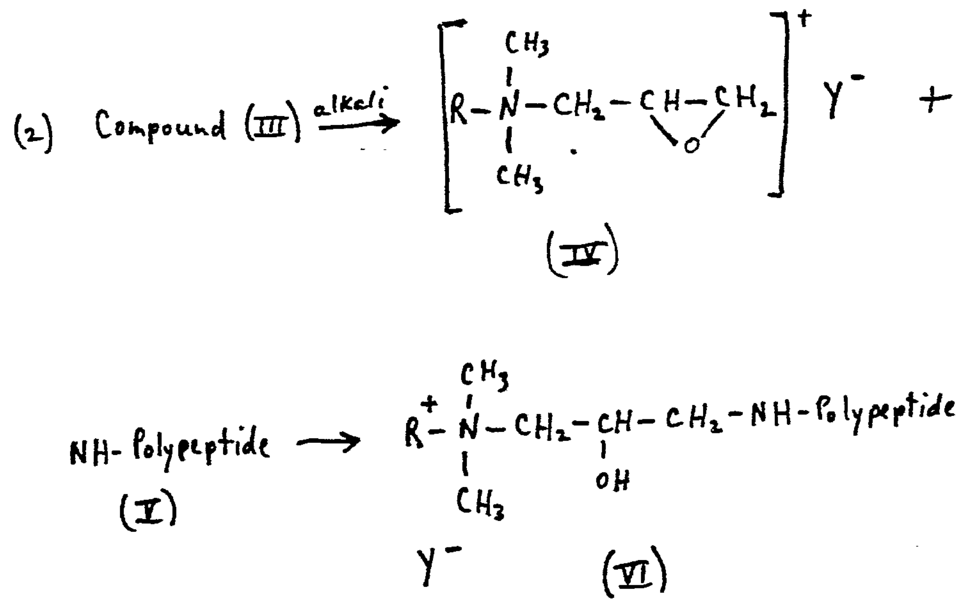


X = chloride or bromide

Y = chloride, nitrate or sulfate

R = $C_{12}H_{25}$, $C_{14}H_{29}$ or $R'CONH(CH_2)_3$, where

$R' = C_{12}H_{25}$, or $C_{14}H_{29}$

The reaction mixture of equation (1) is heated to a temperature of about 50 to about 100 degrees C., preferably to 50 degrees C., for a period of from about 2 to about 5 hours. The reaction mixture is cooled to ambient room

temperature. Sufficient alkali is added to the reaction mixture of equation (2) to maintain a pH value of about 9 to about 11 preferably at a value of about 10. Suitable alkalis include sodium or potassium hydroxide. The protein hydrolysate is then added.

The reaction mixture is reheated to a temperature of about 70 to about 100 degrees C., preferably to about 70 degrees C. The temperature of the reaction mixture and its pH value are continuously maintained until the reaction is complete, as determined by the conventional Ninhydrin Test. When a negative Ninhydrin reaction is achieved, all primary terminal amine groups in the polypeptide chain have bonded to the quaternerized linking agent.

After the reaction is complete, the reaction is cooled to ambient room temperature and neutralized with acid to a pH value of about 5 to about 7, preferably to a value of about 6, for a period of from about 2 to about 5 hours. Suitable acids are illustrated, without limitation, by hydrochloric, sulfuric, or organic acids, such as citric, and lactic acid.

The compounds prepared according to this invention are water soluble in all proportions giving clear solutions, thereby requiring no further filtration or purification. The compounds do not precipitate on standing over extended periods of time thereby showing a high tolerance for electrolyte salts such as sodium chloride. The viscosity of the aqueous products be varied by varying the length of the polypeptide chain and/or the length of the lipophilic chain. If a lipophilic chain of 16 or more carbon atoms is desired, the reaction may be

carried out using a combination of solvents, such as water and a water-miscible organic solvent. Suitable water-miscible organic solvents include, without limitation, alcohols, such as methanol, ethanol, and isopropanol, and dioxan.

While several types of protein hydrolysates are useful herein, two kinds of commercially available protein hydrolysates are used for purposes of illustrating this invention. These materials are sold under the tradenames, Lexein X-250, and Lexein LP-170, by the Inolex Chemical Company.

Lexein X-250 is a polypeptide derivative obtained from enzymatically hydrolyzed animal collagen protein having an average molecular weight of about 2000. The polypeptide material is a liquid extract prepared according to the method described in U.S. Patent No. 3,738,913, containing 55 weight percent solids and a Formol Nitrogen Value of about 11. Lexein LP-170 is a polypeptide material derived from leather scraps chemically hydrolyzed with calcium hydroxide. This liquid extract has a solids content of about 45 weight percent, an average molecular weight of about 500, and a Formol Nitrogen Value of about 17. A typical amino acid distribution of the Lexein materials is demonstrated by the amino acid composition of Lexein X-250 shown below:

Typical amino acid composition of Lexein X250

| Amino Acid | Grams Amino Acid per 100 Gram Hydrolyzed Protein |
| --- | --- |
| Glycine | 25.2 |
| Proline | 13.9 |
| Alanine | 9.5 |
| Arginine | 9.0 |
| Glutamic Acid | 7.4 |
| Hydroxyproline | 10.0 |
| Aspartic Acid | 5.7 |
| Serine | 3.4 |
| Lysine | 4.2 |
| Leucine | 3.2 |
| Valine | 2.4 |
| Threonine | 1.9 |
| Phenylalanine | 1.8 |
| Isoleucine | 1.2 |
| Histidine | 0.8 |
| Methionine | 0.8 |
| Tyrosine | 0.5 |
| Cystine | 0.0 |

It should be noted that proteinaceous materials having a Formol Nitrogen Value of about 8 to about 17 derived from collagen-containing materials, such as the Lexeins, are particularly useful in cosmetic and pharmaceutical compositions. As can be seen from the amino acid distribution of Lexein X-250, the polypeptides have a low sulfur content thereby making them substantially free of objectionable odor, low in color, and low in ash.

The quaternary ammonium derivative of polypeptide may also be prepared from protein

hydrolysates having different degrees of hydrolysis. If desired, a monopeptide, or single amino acid may be used instead of the polypeptide. The amino acid may be a biological, or non-biological amino acid. For example, the amino acid may be N-methyl glycine.

It has now been found that cationic quaternary ammonium derivatives of polypeptides prepared according to the method of this invention, have characteristics making them suitable for use in cosmetic, household and pharmaceutical compositions.

For purposes of illustrating this invention, the quaternary ammonium derivatives of polypeptide described are those obtained by using epichlorohydrin as the epihalohydrin which provides the linking structure. All illustrative compounds, therefore, contain a 2-hydroxypropylene link between the specific polypeptide derivative end group and the cationic lipophilic derivative end group hereinafter identified.

The compounds of this invention have a relatively low Primary Irritation Index (PII) value. For example, the compound of dimethylaminopropylcocamide and Lexein X-250 linked through a structure derived from epichlorohydrin has a PII values at 33 weight percent solids between 2 and 3. For comparison, the PII value of anionic surfactants including a hydrolyzed protein derivative, such as coco fatty acid protein hydrolysate at 30 weight percent solids, is 0.955. A PII value of 2.0, or lower, denotes a mildly irritating material. For use in cosmetic and pharmaceutical compositions, a low primary irritation index is highly desirable for products being applied directly to the skin. A low level of skin irritation is also desirable for household products that come in contact with the

skin, such as dishwashing liquids, hand cleansers, and liquid soaps.

The compounds produce stable-foam lathers making them suitable as foam boosters and surface-active agents. The surface-active properties of the compounds are illustrated by the foaming data shown in Table 1. More particularly, the compounds present in amounts of between 2 and about 5 weight percent, give clear solutions in combination with amphoteric surfactants to provide a very mild foaming detergent suitable as a hair and skin shampoo. Suitable amphoterics include betaine, betaine derivatives, imidazoline derivatives and salts thereof. A specifically useful betaine derivative is cocamido propylbetaine, sold under the tradename, LEXAINE C, by the Inolex Chemical Company. A preferred imidazoline derivative is coco-amphocarboxy-glycinate sold under the tradename LEXOTERIC 2 by Inolex Chemical Company.

The compounds may, if desired, be combined with other cationic surface-active agents, nonionic surface-active agents, and mixtures thereof. It is well known in the art that cationic surfactants may complex with conventional anionic surfactants to produce insoluble products. Thus, appropriate solubilizing agents, known in the art, may be needed for shelf-stability if the compounds are used in combination with such anionic surfactants.

A determination of the isoionic point value of a quaternary ammonium derivative of polypeptide of this invention typically gives an average of about 12, as compared to about 5 to about 6 for the parent protein hydrolysate. This value shows that the compounds prepared in the manner described are positively charged, i.e., cationic. The isoionic

point value was determined using Amberlite MB-1, characterized by its manufacturer, Rohm & Haas, a monobed resin mixture of strongly acidic and strongly basic resins. Cationic quaternary ammonium derivatives of polypeptide present in amounts of between about 1 to about 5 weight percent may be useful as substantive conditioners, and antistatic finishing agents, particularly for natural keratinous fibers, in creme rinse products for hair and in fabric softeners.

A particularly desirable characteristic of protein hydrolysates derived from collagen-containing proteins is their ability to adsorb onto keratinous fibers, especially human hair. Absorption, as used herein, denotes the substantivity of a quaternary ammonium derivative of polypeptide, as determined by the amount of hydroxy proline present on the hair. Hydroxy proline is an amino acid that is not present in untreated hair or in proteinaceous material derived from sources other than collagen-containing materials.

A compound of this invention has a generally increased level of substantivity to human hair, more particularly to hair that has been previously treated with chemical waving and bleaching agents, as compared to the substantivity of the parent protein hydrolysate, as shown by the data in Table 2 and Table 3. Thus, cationic quaternary derivatives of polypeptide are suitable as conditioners in hair waving, and hair dyeing products, including hair bleaches, as well as in hair-conditioner treatments, hair grooming compositions, and like hair-care products.

Another characteristic of cationic quaternary ammonium compounds useful in cosmetics and

topical pharmaceuticals is their ability to inhibit the growth of some bacteria species. The anti-microbial properties of the quaternary ammonium derivatives of polypeptide disclosed are illustrated by the compound of N,N-dimethylcocamidopropylamine and Lexein X250 linked through a structure derived from epichlorohydrin, at a concentration of 10 weight percent. The compound inhibited the growth of Gram (+) Staphylococcus epidermis (ATCC 1228) and Bacillus subtilis (ATCC 1633) and showed with some inhibition against the Gram (-) Eschericia coli (ATCC 25922). Anti-microbicidal activity was determined by using the well-known qualitative Zone of Inhibition Method. For the test, a blank sensitivity test disc (1/4 inch diameter) is dipped into a test solution and is then placed on a petri dish previously streaked with a test culture. The test plate is incubated at 35 degrees C. for twenty-four hours and then read for the presence or absence of a zone of inhibition; i.e. area of no growth.

Compounds prepared according to this invention are soluble in water, or in water-organic solvent mixtures, compositions, such as emulsion lotions, creams, and the like, typically used in cosmetic and pharmaceutical products intended for topical use on skin or hair. The compounds of this invention may further be used as conditioners, and anti-static agents for hair-care products and fabric softeners, as skin-protective surface-active agents in liquid hand soaps and in liquid dish-washing detergents and as bactericidal agents in skin-care products. Auxiliary ingredients may be included selected from known materials for their usual purposes, such as coloring agents, fragrances, stabilizers, thickeners, and the like.

The invention is further illustrated in the following examples, which are not intended to be limiting.

Example 1: Preparation of Quaternary Ammonium Derivative of Polypeptide.

To 130 milliliters of water is added 23 grams of concentrated hydrochloric acid (36-38 weight percent) with stirring. N,N-dimethyldodecylamine, in an amount of 52 grams (0.232 molar), is then slowly added to the acidified water with stirring to dissolve the amine. Subsequently, epichlorohydrin is added to this mixture in an amount of 21.5 grams (0.232 molar). The entire mixture is heated to a temperature of 50 degrees C. and so maintained for a period of two hours.

The mixture is cooled to room temperature, and its pH value is adjusted to a stable value of 10 by titrating it with a solution of 25 weight percent sodium hydroxide. The reaction mixture is stirred for an additional 1-1/2 hours at room temperature. An enzymatically hydrolyzed protein polypeptide solution (Lexein X-250) having a solids content of 55 weight percent in water, and a Formol Nitrogen Value of about 11, is added in an amount of 98 grams as is. Additional water in an amount of 35 milliliters is added to the reaction mixture and its pH value readjusted to 10 as needed.

The reaction mixture is then heated to 70 degrees C. and is so maintained for a period of three hours, or until a negative Ninhydrin test reaction is achieved. During this period, the pH of the mixture is also monitored and readjusted, as needed, to maintain it at a value of 10. When the reaction is complete, the mixture is cooled to room temperature

and then neutralized to a stable pH value of 6 by titrating it with a 36% hydrochloric acid.

The compound obtained is yellow in color, and gives a negative Ninhydrin reaction.

Example 2: Preparation of Quaternary Ammonium Derivative of Polypeptide

The procedure of Example 1 is followed except that the hydrolyzed protein polypeptide used is a chemically hydrolyzed collagen protein (Lexein LP-170) having a solids concentration of 45 weight percent in water, and a Formol Nitrogen Value of about 17, and is added in an amount of 70 grams (as is). A similar yellow, compound having a bland odor and negative Ninhydrin reaction is obtained.

Example 3: Preparation of Quaternary Ammonium Derivative of Polypeptide

To 75 grams of water is added 7.5 grams of concentrated hydrochloric acid (36-38 weight percent) with stirring. N,N-dimethyltetradecylamine, sold under the tradename, Armeen DM14D, by Armak Company, is slowly added in an amount of 19 grams (0.075 molar), to the acidified water with stirring to dissolve the amide. To this mixture is added epichlorohydrin in an amount of 6.9 grams (0.075 molar) and the entire mixture is heated to a temperature of 50 degrees C. and so maintained for a period of two hours.

The mixture is cooled to room temperature and its pH value is adjusted to a stable value of 10 by titrating it with a solution of 25 weight percent sodium hydroxide. The reaction mixture is stirred for an additional 1-1/2 hours at room temperature. The enzymatically hydrolyzed protein polypeptide,

Lexein X-250, of Example 1 is added in an amount of 32 grams (as is) along with additional water in an amount of 50 milliliters to the reaction mixture and its pH value readjusted to 10 as needed.

The reaction mixture is then heated to 70 degrees and is maintained for three hours [or until a negative Ninhydrin test reaction is achieved]. During this period the pH of the mixture is also monitored and readjusted as needed, to maintain it at a value of 10. When the reaction is complete, the mixture is cooled to room temperature and then neutralized to a stable pH value of 6 by titrating it with a 36% solution of hydrochloric acid.

The compound obtained is yellow in color, and gives a negative Ninhydrin reaction.

Example 4: Preparation of Quaternary Ammonium Derivative of Polypeptide

The procedure of Example 3 is followed except that the hydrolyzed protein polypeptide, Lexein LP-170, of Example 2 is added in an amount of 23 grams (as is). A similar yellow compound and negative Ninhydrin reaction is obtained.

Example 5: Preparation of Quaternary Ammonium Derivative of Polypeptide

To 240 grams of water is added 23 grams of concentrated hydrochloric acid (36-38 weight percent) with stirring. To this solution is slowly added N,N-dimethylcocamidopropylamine, sold under the tradename, Lexamine C-13, by the Inolex Chemical Company, in an amount of 71 grams (0.232 molar) with stirring to dissolve. Subsequently, epichlorohydrin is added to the mixture in an amount of 21.5 grams (0.232 molar) The entire mixture is heated to a temperature of 50 degrees C. and is so maintained for a period of two hours.

The mixture is cooled to room temperature and its pH value is adjusted to a stable value of 10 by titrating it with a solution of 25 weight percent sodium hydroxide.

The reaction mixture is stirred for an additional 1-1/2 hours at room temperature. The hydrolyzed protein polypeptide, Lexein X-250, of Example 1 in an amount of 124 grams (as is) is added to the reaction mixture and its pH value readjusted to 10 as needed.

The reaction mixture is then heated to a temperature of 70 degrees C. and is so maintained for three hours [or until a negative Ninhydrin reaction is achieved]. During this period, the pH of the mixture is also monitored and readjusted, as needed, to maintain it at a value of 10. When the reaction is complete, the mixture is cooled to room temperature and then neutralized to a stable pH value of 6 by titrating it with a 26% solution of hydrochloric acid.

The compound obtained is yellow in color, has a bland odor, and gives a negative Ninhydrin reaction.

Example 6: Preparation of Quaternary Ammonium Derivative of Polypeptide

The procedure of Example 5 is followed, except that the hydrolyzed protein polypeptide, Lexein LP-170, of Example 2 having a solids content of [47] weight percent in water, is added in an amount of 70 grams (as is). A similar yellow, compound having a bland odor and negative Ninhydrin reaction is obtained.

Example 7: Preparation of Quaternary Ammonium Derivative of Polypeptide

To 230 milliliters of water is added 23 grams of concentrated hydrochloric acid (36-38 weight percent) with stirring. To this solution is slowly added N,N-dimethyldodecamidopropylamine, sold under the tradename, Lexamine L-13, by the Inolex Chemical Company, in an amount of 66 grams (0.232 molar), with stirring to dissolve the amide. To this mixture is added epichlorohydrin in an amount of 21.5 grams (0.232 molar). The entire mixture is heated to a temperature of 50 degrees C. and is so maintained for a period of two hours. The mixture is cooled to room temperature and then neutralized to a stable pH value of 10 by titrating it with a 25% solution of sodium hydroxide.

The reaction mixture is stirred for an additional 1-1/2 hours. The hydrolyzed protein polypeptide, Lexein X-250, of Example 1 is added in an amount of 124 grams (as is) to the reaction mixture and its pH value readjusted to 10 as needed.

The reaction mixture is then heated to a temperature of 70 degrees C. and is so maintained for four hours [or until a negative Ninhydrin test reaction is achieved]. During this period, the pH of the mixture is also monitored and readjusted, as needed, to maintain it at a value of 10. When the reaction is complete, the mixture is cooled to room temperature and then neutralized to a stable pH value of 6 by titrating it with concentrated hydrochloric acid.

The compound obtained is yellow in color, has a bland odor, and gives a negative Ninhydrin reaction.

Example 8: Preparation of Quaternary Ammonium Derivative of Polypeptide

The procedure of Example 7 is followed, except that the hydrolyzed protein polypeptide, Lexein LP-170, of Exammple 6 is used added in an amount of 70 grams (as is). A similar yellow compound having a bland odor and negative Ninhydrin reaction is obtained.

Example 9: Preparation of Quaternary Ammonium Derivative of Polypeptide

To 240 milliliters of water is added 23 grams of concentrated hydrochloric acid (36-38 weight percent) with stirring. To this solution is slowly added N,N-dimethyltetradecamidopropylamine, sold under the tradename, Lexamine M-13, by the Inolex Chemical Company, in an amount of 73 grams (0.232 molar) with stirring to dissolve the amide. To this mixture is added epichlorohydrin in an amount of 21.5 grams (0.232 molar). The entire mixture is heated to a temperature of 50 degrees C. and is so maintained for a period of two hours [or until clear]. The mixture is cooled to room temperature and then neutralized to a stable pH value of 10 by titrating it with a 25% solution of sodium hydroxide.

The reaction mixture is stirred for an additional 1-1/2 hours. The hydrolyzed protein polypeptide, Lexein X-250, of Example 7 is added in an amount of 100 grams (as is) along with and water in an amount of 230 milliliters to the reaction mixture and its pH value readjusted to 10 as needed.

The reaction mixture is then heated to a temperature of 70 degrees C. for a period of five hours [or until a negative Ninhydrin reaction is achieved]. During this period, the pH of the mixture

is also monitored and readjusted as needed to maintain it at a value of 10. When the reaction is complete, the mixture is cooled to room temperature and then neutralized to a stable pH value of 6 by titrating it with a 36% solution of hydrochloric acid.

The compound obtained is yellow in color, has a bland odor, and gives a negative Ninhydrin reaction.

Example 10: Preparation of Quaternary Ammonium Derivative of Polypeptide

The procedure of Example 9 is followed, except that the hydrolyzed protein polypeptide, Lexein LP-170, of Example 8 is added in an amount of 70 grams (as is) along with additional water in the amount of 120 milliliters. A similar yellow, compound having a bland odor and negative Ninhydrin reaction is obtained.

Example 11: Surface-active Properties of Quaternary Ammonium Derivatives of Polypeptides

The surface-active properties of the compounds prepared according to the procedures of Examples 1-10 were determined on the basis of the amount of foam they generate at a temperature of 25 degrees C. using the well-known Ross-Miles method (ASTM D1173). For the test, an aqueous solution having a solids content of 0.6 weight percent compound in deionized water was prepared and adjusted to a pH value of about 7 by adding sodium hydroxide. The height of the foam column generated was measured initially. The stability of the foam was determined by remeasuring the foam column after a standing drain period of about 5 minutes. The foaming data are shown in Table 1.

Table 1
Surface-Active Properties of
Quaternary Ammonium Derivatives of Polypeptide

| The Quaternary Ammonium Derivative of Polypeptide at 0.6 weight percent solids | Measured Foam Height in Milliliters | |
|---|---|---|
| | Initial | After 5 Min. |
| Compound of Example 1 | 245 | 230 |
| Compound of Example 2 | 210 | 195 |
| Compound of Example 3 | 215 | 210 |
| Compound of Example 4 | 220 | 220 |
| Compound of Example 5 | 220 | 220 |
| Compound of Example 6 | 210 | 200 |
| Compound of Example 7 | 225 | 220 |
| Compound of Example 8 | 235 | 230 |
| Compound of Example 9 | 215 | 205 |
| Compound of Example 10 | 195 | 190 |

As may be seen from the data, all compounds generated substantial amounts of long-lasting foam.

Example 12: Hair Conditioner Study

The product of Example 10 in sufficient quantity to provide 2.0 parts of the quaternary ammonium derivative of the polypeptide was blended with 0.15 parts of methylparaben and water (qs to 97.95 parts) to make up part A of a hair conditioner composition. Part B of the composition was a mixture of 1.0 parts of cetyl alcohol, 1.0 parts of stearyl alcohol and 0.05 parts of propylparaben.

Parts A and B were each heated to 70°-75° C. and part B was then added to part A with constant stirring to produce a hair conditioning product.

The hair conditioning product was applied by an experienced operator to the hair of eight women on the same day and then to the hair of seven women a week later for a total of fifteen applications. Six of the heads treated on the second week were heads that had been treated on the first week.

The operator was asked to evaluate, on a scale of 1 to 5 (with 5 being best), eight different characteristics of hair condition. The average ratings for the fifteen applications were as follows:

| | |
|---|---|
| Detangling | 4.7 |
| Wet Combability | 4.2 |
| Dry Combability | 3.6 |
| Absence of Static | 4.9 |
| Curl Bounce | 3.6 |
| Fullness | 3.8 |
| Smoothness | 3.3 |
| Shine | 3.4 |

In addition, the operator noted that her hands, after contact with the hair conditioning product, were very smooth and soft.

Example 13: Substantivity Properties of Quaternary Ammonium Derivatives of Polypeptides

The substantivity of the compounds prepared according to Examples 1-10 to human hair was determined by measuring the amount of hydroxyproline present in the compound and in the hair treated with same. The hydroxyproline content of the compound was analyzed according to the method of S. A. Karjala et al. J. Soc. Cosmet. Chem., 17, 513 (1966). Table 2 shows the weight percent of hydroxyproline present in the compounds prepared according to Examples 1-10 expressed on an as-is basis, and on a weight percent dry basis calculated from the hydroxy proline factor of the parent collagen protein: i.e.; protein hydrolysate.

Table 2

Hydroxy Proline Content of Quaternary Ammonium Derivatives of Polypeptide

| Quaternary Ammonium Derivative of Polypeptide | Weight Percent Solids (As is) | Weight Percent Hydroxy-Proline | Percent Percent Hydrolyzed Protein (a) |
|---|---|---|---|
| Compound of Example 1 | 38.5 | 1.54 | 40.00 |
| Compound of Example 2 | 39.9 | 1.21 | 24.26 |
| Compound of Example 3 | 33.7 | 1.09 | 32.20 |
| Compound of Example 4 | 27.0 | 0.77 | 22.80 |
| Compound of Example 5 | 33.0 | 1.37 | 41.50 |
| Compound of Example 6 | 27.9 | 0.61 | 17.50 |
| Compound of Example 7 | 34.1 | 1.57 | 46.00 |
| Compound of Example 8 | 29.1 | 0.80 | 22.00 |
| Compound of Example 9 | 19.6 | 0.80 | 40.80 |
| Compound of Example 10 | 19.4 | 0.50 | 20.60 |

(a) $\frac{\text{weight percent hydroxyproline} \times PF \times 100}{\text{weight percent solids (as is)}}$

where PF is the weight percent value of hydroxyproline in the parent hydrolyzed collagen protein. For compounds of Examples 1, 3, 5, 7 and 9, the PF value is 10; for compounds of Examples 2, 4, 6, 8, and 10, the PF value is 8.

Each of the aqueous compounds shown in Table 2 was diluted with the appropriate amount of water needed to bring the solids to a concentration of 5 weight percent. Swatches of human hair, each weighing about 0.5 grams, that had been previously bleached and chemically waved, were obtained from De Meo Brothers of New York. The bleached-waved hair swatches were cleaned by shampooing them with a solution containing one weight percent of ethoxylated alkyl phenol (adopted CTFA name: octoxynol-9), sold under the tradename, Triton X-100, by Rohm and Haas Company, and thereafter rinsing and drying. A clean tress was soaked in about 50milliliters of the aqueous composition for ten minutes, rinsed for

thirty seconds with warm tap water and then heat dried.

The substantivity of the quaternary ammonium derivative of polypeptide was determined by analyzing fiber samples taken from the treated hair for hydroxyproline content according to aforementioned Karjala et al. method. The weight percent solids of the cationic quaternary ammonium derivative of polypeptide present on hair; i.e. cationic compound, are shown in Table 3 along with the calculated weight percent solids of hydoxy proline present in the aqueous treating solution, and calculated weight percent solids of hydroxyproline present on hair.

Table 3
Substantivity of Quaternary Ammonium Derivatives of Polypeptides To Bleached-Waved Hair

| Quaternary Ammonium Derivative of Polypeptide | Weight % Solids Hydroxyproline Test Solution (a) | Percent Hydrolyzed Portion On Hair | Weight % Cationic Compound on Hair (b) |
|---|---|---|---|
| Composition of Example 1 | 0.035 | 0.35 | 0.86 |
| Composition of Example 2 | 0.016 | 0.13 | 0.53 |
| Composition of Example 3 | 0.030 | 0.30 | 0.93 |
| Composition of Example 4 | 0.018 | 0.14 | 0.61 |
| Composition of Example 5 | 0.021 | 0.21 | 0.50 |
| Composition of Example 6 | 0.013 | 0.10 | 0.57 |
| Composition of Example 7 | 0.042 | 0.42 | 0.91 |
| Composition of Example 8 | 0.070 | 0.16 | 0.73 |
| Composition of Example 9 | 0.036 | 0.36 | 0.88 |
| Composition of Example 10 | 0.020 | 0.16 | 0.78 |
| For Comparison: | | | |
| Lexein X-250 (Parent polypeptide of Examples 1,3,5,7,9) | 0.086 | 0.86 | ----- |
| Lexein LP-170 (Parent polypeptide of Examples 2,4,6,8,10) | 0.060 | 0.48 | ------ |

(a) The test solution contains 5.0 weight percent solids of test material.

(b) percent hydrolyzed portion X100 weight percent hydrolyzed protein.

The present invention has been described with respect to preferred embodiments. It will be clear to those skilled in the art that modifications and/or variations of the disclosed compositions and method can be made without departing from the scope of the invention set forth herein. The invention is defined by the claims that follow.

WHAT IS CLAIMED IS:

1. A water soluble, cationic quaternary ammonium derivative of a polypeptide having the formula:

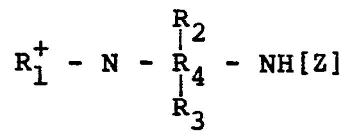

wherein $R_1$ is selected from at least one $C_{12}$ to $C_{22}$ alkyl radical and carboxamide radicals of the formula $R_5-\overset{O}{\overset{\|}{C}}-NH-R_6$ - wherein $R_5$ is selected from at least one $C_{12}$ to $C_{22}$ alkyl radical and $R_6$ is selected from at least one $C_2$ to $C_3$ alkylene radical, where $R_2$ is a $C_1$ to $C_4$ alkyl radical and $R_3$ is selected from the group consisting of $C_1$ to $C_4$ radicals and at least one $C_{12}$ to $C_{22}$ alkyl radical; $-R_4-$ is a linking structure derived from an epihalohydrin, where the halogen atom comprises chlorine and bromine; and -NH[Z] is an amino acid segment containing from 1 to 500 amino acid units.

2. The cationic quaternary ammonium derivative of claim 1 wherein the $R_1$ comprises a $C_{12}$ to $C_{14}$ alkyl radical and $R_2$ amd $R_3$ are methyl radicals.

3. The cationic quaternary ammonium derivative of claim 1 wherein $R_2$ and $R_3$ are methyl radicals and $R_1$ is a carboxamide radical comprising a $C_{12}$ to $C_{14}$ alkyl radical and a $C_3$ alkylene radical.

4. The quaternary ammonium derivative of claim 1 wherein the linking structure derived from

epihalohydrin is derived from epichlorohydrin and is represented by $-CH_2-\underset{\mid \atop OH}{CH}-CH_2-$.

5. The quaternary ammonium derivative of claim 1 wherein [Z] is a polypeptide derivative group derived from hydrolyzed animal collagen protein and has a Formol Nitrogen Value of between about 8 and about 17, and comprises from about 3 to about 30 amino acid units in a polypeptide chain.

6. The quaternary ammonium derivative of claim 1 wherein the hydrophilic polypeptide derivative group is derived from chemically hydrolyzed animal collagen protein and has an average Formol Nitrogen Value of about 11, and an average molecular weight of about 500.

7. The quaternary ammonium derivative of claim 1 in which the compound comprises the reaction product of a fatty tertiary amine having at least one $C_{12}$ to $C_{14}$ alkyl radical group in the fatty chain, epichlorohydrin, and a protein hydrolysate radical derived from hydrolyzed animal collagen protein.

8. A method of preparing the cationic quaternary ammonium derivative of claim 1 which comprises the steps of

(a) reacting an amine compound of the class consisting of tertiary amines and salts thereof, with an epihalohydrin compound in the presence of water;

(b) reacting the aqueous reaction mixture of step (a) with an aqueous solution containing a compound of from 1 to 500 amino acid units to form a cationic quaternary ammonium derivative of the amino acid compound;

said tertiary amine having the formula:

$$R_1 - \underset{R_3}{\overset{R_2}{N}}$$

wherein $R_1$ is selected from at least one $C_{12}$ to $C_{22}$ alkyl radical and carboxamide radicals of the formula

$$R_5 - \overset{O}{\overset{\|}{C}}-NH-R_6$$

wherein $R_5$ is selected from at least one $C_{12}$ to $C_{22}$ alkyl radical and carboxamide $R_6$ is selected from at least one $C_2$ to $C_3$ alkylene radical, wherein $R_2$ is a $C_1$ to $C_4$ alkyl radical and $R_3$ is selected from the group consisting of $C_1$ to $C_4$ radicals and $C_{12}$ to $C_{22}$ alkyl radicals.

9. The method of claim 8 wherein said amino acid compound is a polypeptide derived from protein hydrolysis.

10. The method of claim 9 wherein the reaction of step (a) is carried out at a temperature of between about 50 to about 100 degrees C. and the reaction of step (b) includes the presence of alkali and is carried out at a temperature of between about 70 and about 100 degrees C. for a period of time sufficient to react all the amine groups in the amino acid units in the polypeptide chain.

11. The method of claim 9 wherein the reaction mixture in step (a) includes a water-miscible organic solvent.

12. The method of claim 9 wherein a fatty tertiary amine salt, having at least one $C_{12}$ to $C_{14}$ alkyl radical group in the fatty chain, is reacted with epichlorohydrin in water in step (a), said reaction being carried out at a temperature of about 50 degrees C.; and wherein the hydrophilic

polypeptide in step (b) comprises a protein hydrolysate radical derived from hydrolyzed animal collagen protein, said reaction being carried out in the presence of sufficient alkali to adjust the pH value of the reaction mixture to about 10, said reaction being further carried out at a temperature of about 70 degrees C.

13. A complex of N,N-dimethylcocamido-propylamine with an enzymatically hyrolyzed protein polypeptide having a Formol Nitrogen Value of about 11, said N,N-dimethylcocamidopropylamine and said polypeptide being linked through a 2-hydroxypropylene group.

14. A complex of N,N-dimethyltetradecamido-propyl amine with a chemically hydrolyzed protein polypeptide having a Formol Nitrogen Value of about 17, said N,N-dimethyltetradecamidopropylamine and said polypeptide being linked through a 2-hydroxy propylene group.

European Patent Office

**EUROPEAN SEARCH REPORT**

0109074

Application number

EP 83111309.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,Y | PATENT ABSTRACTS OF JAPAN, unexamined applications, C section, vol. 3, no. 32, March 17, 1979<br>THE PATENT OFFICE JAPANESE GOVERNMENT<br>page 133 C 40<br>* Kokai-no. 54-8 688 (LION YUSHI K.K.) *<br>-- | 1,4-6, 8-10 | C 07 C 103/52<br>C 07 C 103/54<br>C 07 C 101/24<br>C 07 G 7/00//<br>A 61 K 37/02<br>A 61 K 7/06 |
| D,Y | PATENT ABSTRACTS OF JAPAN, unexamined applications, C section, vol. 3, no. 32, March 17, 1979<br>THE PATENT OFFICE JAPANESE GOVERNMENT<br>page 137 C 40<br>* Kokai-no. 54-8 728 (LION YUSHI K.K.) *<br>-- | 1,4-6, 8-10 | **TECHNICAL FIELDS SEARCHED (Int. Cl. 3)** |
| D,Y | FR - A - 1 149 161 (SINNOVA)<br>* Pages 1,2; claims 1-6 *<br>-- | 1-3,8-10 | C 07 C 103/00<br>C 07 C 101/00<br>C 07 G 7/00<br>C 12 P 21/00 |
| Y | DE - A - 1 959 651 (HENKEL & CIE GMBH)<br>* Pages 1-5; claims 1-4 *<br>-- | 1,5,6, 8-10 | |
| Y | DE - A - 2 126 397 (HENKEL & CIE GMBH)<br>* Pages 1-5; claim *<br>-- | 1,5,6, 8-10 | |

The present search report has been drawn up for all claims

| Place of search | Date of completion of the search | Examiner |
|---|---|---|
| VIENNA | 20-02-1984 | PETROUSEK |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

European Patent Office

**EUROPEAN SEARCH REPORT**

0109074

Application number

EP 83111309.7

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| Y | DE - A - 2 151 739 (HENKEL & CIE GMBH)<br>* Pages 1-4; claims 1-3 *<br>-- | 1,5,6, 8-10 | |
| A | EP - A2 - 0 020 907 (TH. GOLD-SCHMIDT AG)<br>* Claim 1 *<br>-- | 1-3,8 | |
| D,A | US - A - 3 738 913 (V.L. JOHNSON et al.)<br>* Columns 2,4; claim 1 *<br>---- | 1,5,6, 8,9 | TECHNICAL FIELDS SEARCHED (Int. Cl.3) |

EPO Form 1503.2 06.78